Europäisches Patentamt

European Patent Office

Office européen des brevets

(11) Publication number: **0 162 705 B1**

## EUROPEAN PATENT SPECIFICATION

(45) Date of publication of patent specification: **29.05.91**     (51) Int. Cl.⁵: **A61K 9/08**, A61K 47/00, A61K 31/44

(21) Application number: **85303588.9**

(22) Date of filing: **21.05.85**

(54) **Injection of nicardipine hydrochloride and production thereof.**

(30) Priority: **22.05.84 JP 102991/84**

(43) Date of publication of application:
**27.11.85 Bulletin 85/48**

(45) Publication of the grant of the patent:
**29.05.91 Bulletin 91/22**

(84) Designated Contracting States:
**AT BE CH DE FR GB IT LI NL SE**

(56) References cited:
**EP-A- 0 117 888**
**EP-A- 0 126 315**
**EP-A- 0 131 228**
**FR-A- 2 020 684**

**PATENTS ABSTRACTS OF JAPAN, vol. 8, no.
85 (C-219)[1522], 18th April 1984; & JP-A-59
7163 (YAMANOUCHI SEIYAKU K.K.)
14-01-1984**

(73) Proprietor: **YAMANOUCHI PHARMACEUTICAL
CO. LTD.**
**No. 3-11 Nihonbashi-Honcho, 2-chome Chuo-
ku**
**Tokyo(JP)**

(72) Inventor: **Katayasu, Ogawa**
**No. 2735-78, Ohaza Kasahata aza Ohkasa
Kawagoe-shi Saitama(JP)**
Inventor: **Ohtani, Go**
**No. 4-25-2, Funabashi
Setagaya-ku Tokyo(JP)**
Inventor: **Yokota, Shoji**
**No. 3-16-1, Hasune
Itabashi-ku Tokyo(JP)**
Inventor: **Aruga, Masayoshi**
**No. 51614, Midorigaoka
Ageo-shi Saitama(JP)**

(74) Representative: **Geering, Keith Edwin et al
REDDIE & GROSE 16 Theobalds Road
London WC1X 8PL(GB)**

**Description**

This invention relates to an injection of nicardipine hydrochloride having cerebral vascular dilator activity, coronary dilator activity, and anti-hypertension activity; and to a process of producing the injection.

Hitherto, oral administration formulations containing nicardipine hydrochloride have been available, but no satisfactory injection containing nicardipine hydrochloride has been developed. This is because the compound has reduced solubility in known isotonic aqueous injection media; thus in the presence of sodium chloride, which is usually used as an isotonizing agent for injections, it is only possible to obtain an aqueous nicardipine hydrochloride solution which is of too low concentration and/or of insufficient stability for satisfactory use as an injection.

We have now found that when nicardipine hydrochloride is dissolved in water together with 2 to 7 w/v% of polyhydric alcohol, a stable isotonic aqueous solution of the nicardipine hydrochloride is unexpectedly obtained.

According to this invention there is provided an injection of nicardipine hydrochloride containing 2 to 7 w/v% of polyhydric alcohol and preferably having a pH of 2.5 to 5.5.

This invention also provides a process of producing an injection of nicardipine hydrochloride which comprises dissolving in water nicardipine hydrochloride and 2 to 7 w/v% of polyhydric alcohol based on the total injection, and if necessary adjusting the pH of the solution to 2.5 to 5.5, preferably 2.5 to 5.0. Even when the pH of the solution as initially formed is from 2.5 to 5.0 or 5.5, it may be preferred to adjust the pH to a specific desired value, e.g. 3.0, 3.5 or 5.0.

As polyhydric alcohols for use in this invention, there are e.g. sorbitol, mannitol, xylitol, propylene glycol, glycerol, inositol. They may be used alone or in admixture of any two or more. The polyhydric alcohol constitutes about 2 to 7, preferably about 5, w/v% of the whole amount of the injection. If the polyhydric alcohol should give insufficient isotonization when used at about 2 w/v%, the isotonization of the injection may be controlled by using other isotonizing agent.

Also, if the pH of the injection is lower than 2, the stability of nicardipine hydrochloride is reduced, while if the pH is higher than 6, the solubility of nicardipine hydrochloride is reduced.

The concentration of nicardipine hydrochloride in the injection of this invention is dependent upon the polyhydric alcohol concentration and the pH. By selecting appropriate conditions, a solubility of about 0.6 w/v% can be obtained.

The injection of this invention can be prepared by dissolving predetermined amounts of nicardipine hydrochloride and polyhydric alcohol in water, e.g. at 50°C to 60°C, adjusting the pH (if necessary or desired), preferably to about 3.5, and then adjusting the volume of the solution to a predetermined volume by the addition of water. The pH of the solution can be controlled by mineral acid such as hydrochloric acid or, as the case may be, by base such as sodium hydroxide or sodium hydrogencarbonate.

The injection of nicardipine hydrochloride thus obtained can maintain its desired concentration (higher than 0.1 w/v%) and can be stably stored for a long time without change in quality. In addition, it is preferred to use light-resistant ampoules for the injection.

Tests on the solubility if nicardipine hydrochloride and the stability of nicardipine hydrochloride solutions are set out below :

A. Effect of polyhydric alcohol

(a) Storing at 60°C

(i) Sample:

The injection of Example 1: Sorbitol (5 w/v%)
The injection of Example 2: Mannitol (5 w/v%)
The injection of Example 3: Xylitol (5 w/v%)
The injection of Example 4: Propylene glycol (5 w/v%)
Control : An aqueous solution of nicardipine hydrochloride (0.1 w/v%, pH 3.5) containing no polyhydric alcohol.

(ii) Storage conditions:

2

Each sample was stored in a bath maintained at 60°C and sampled after the same definite periods.

(iii)Quantitative determination:

The quantitative determination was performed by high performance liquid chromatography (HPLC).

Conditions of HPLC :

Column: Nucleosil $C_{18}$ (150 x 4 mm )
Column temperature: 40°C
Eluent: Methanol-0.01 M potassium hydrogenphosphate solution (18 : 7)
Detection: Ultraviolet absorption detector Wave length 254 n. m.

(iv) Result:

The remaining percentage of nicardipine hydrochloride in each sampled solution is shown in the following table.

| Sample | Storage period (weeks) | | | |
|---|---|---|---|---|
| | 0 | 4 | 8 | 12 |
| Example 1 | 100 | 96.09 | 89.57 | 69.67 |
| " 2 | 100 | 93.08 | 88.26 | 71.63 |
| " 3 | 100 | 95.04 | 85.60 | 69.24 |
| " 4 | 100 | 97.20 | 88.03 | 74.39 |
| Control | 100 | 93.47 | 77.94 | 49.13 |

(b) Storing at 100°C

(i) Sample:

The injection of Example 7: Sorbitol (2 w/v%)
The injection of Example 8: Sorbitol (4 w/v%)
The injection of Example 9: Mannitol (2 w/v%)
The injection of Example 10: Mannitol (4 w/v%)
The injection of Example 11: Xylitol (2 w/v%)
The injection of Example 12: Xylitol (4 w/v%)
The injection of Example 13: Propylene glycol (2 w/v%)
The injection of Example 14: Propylene glycol (4 w/v%)
Control : as for test A (a) above.

(ii) Storage conditions:

Each sample was stored in a chamber at 100°C and sampled after the same definite periods.

(iii)Quantitative determination:

as for test A (a) above.

(iv) Result:

The remaining percentage of nicardipine hydrochloride in each sampled solution is shown in the following table.

| Sample | Storage period (days) | | |
|---|---|---|---|
| | 1 | 3 | 7 |
| Example 7 | 93.18 | 75.77 | 47.45 |
| " 8 | 92.98 | 79.62 | 49.66 |
| " 9 | 90.35 | 74.35 | 47.87 |
| " 10 | 92.43 | 78.27 | 50.03 |
| " 11 | 91.76 | 75.60 | 48.06 |
| " 12 | 94.26 | 78,43 | 50.28 |
| " 13 | 97.06 | 79.87 | 44.58 |
| " 14 | 92.39 | 78.02 | 45.77 |
| Control | 88.69 | 58.26 | 20.48 |

B. Influence of pH

(i) Sample:

The injection of the Comparison example: pH 2.0
The injection of Example 15: pH 3.0
The injection of Example 16: pH 4.0
The injection of Example 17: pH 5.0

(ii) Test conditions:

Each sample was stored in a chamber maintained at $100^\circ$ C and sampled after the same definite periods.

(iii)Quantitative determination:

as for test A (a) above.

(iv) Result:

The remaining percentage of nicardipine hydrochloride in each sampled solution is shown in the following table.

4

| Sample | (pH) | Storage time (hrs.) | | | |
|---|---|---|---|---|---|
| | | 0 | 2.5 | 5 | 10 |
| Comparison example | (2.0) | 100 | 92.02 | 88.10 | 85.53 |
| Example 15 | (3.0) | 100 | 98.87 | 98.74 | 95.14 |
| Example 16 | (4.0) | 100 | 99.18 | 99.68 | 99.73 |
| Example 17 | (5.0) | 100 | 98.36 | 97.23 | 95.40 |

C. Comparative test of stability of sodium chloride/nicardipine hydrochloride solution and polyhydric alcohol/nicardipine hydrochloride solution.

(i) Sample:

1. Nicardipine hydrochloride 1 mg/ml, 5 w/v% sorbitol, pH 3.5
2. " " , 5 w/v% mannitol, "
3. " " , 5 w/v% xylitol, "
4. " " , 5 w/v% propylene glycol, "
5. " " , — , "
6. " about 0.5 mg/ml, 0.9 w/v% NaCl, "

(ii) Storage conditions:

Each sample was stored in a chamber at 121°C for 6 hours.

(iii) Results:

| Sample No. | 1 | 2 | 3 | 4 | 5 | 6 |
|---|---|---|---|---|---|---|
| Remaining percentage | 93.30% | 95.88% | 95.28% | 94.99% | 90.61% | 79.03% |

D. Comparative test of the effects of sodium chloride and polyhydric alcohol on nicardipine hydrochloride solubility.

(i) Solubility of nicardipine hydrochloride in 0.6w/v% sodium chloride solution.

| Storage temp. | Sampling time (hrs) | Solubility (%) | pH |
|---|---|---|---|
| 5°C | 1 | 0.042 | 3.48 |
| | 2 | 0.032 | 3.47 |
| | 4 | 0.043 | 3.50 |
| | 6 | 0.036 | 3.50 |
| | 72 | 0.032 | 3.53 |
| 25°C | 1 | 0.056 | 3.48 |
| | 2 | 0.056 | 3.57 |
| | 4 | 0.057 | 3.59 |
| | 6 | 0.057 | 3.52 |
| | 72 | 0.055 | 3.58 |

(ii) Solubility of nicardipine hydrochloride in 5w/v% sorbitol solution:

| Storage temp. | Sampling time (hrs) | Solubility (%) | pH |
|---|---|---|---|
| 5°C | 1 | 0.408 | 3.36 |
| | 2 | 0.308 | 3.33 |
| | 4 | 0.295 | 3.32 |
| | 6 | 0.270 | 3.30 |
| | 24 | 0.380 | 3.27 |
| | 42 | 0.336 | 3.23 |
| 25°C | 1 | 0.511 | 3.45 |
| | 2 | 0.503 | 3.35 |
| | 4 | 0.471 | 3.70 |
| | 6 | 0.482 | 3.47 |
| | 24 | 0.515 | 3.50 |
| | 72 | 0.522 | 3.52 |

The following Examples are intended to illustrate this invention

Example 1

About 2 litres of distilled water was heated to 50 to 60°C and 2.5 g of nicardipine hdyrochloride and 125 g of sorbitol were dissolved therein with stirring. After cooling the solution (pH about 4.5) thus obtained to room temperature, the pH thereof was adjusted to 3.5 using 0.1 N hydrochloric acid. Then, the whole volume thereof was adjusted to 2.5 litres by the addition of distilled water and after filtering the solution, 5 ml. aliquots of the solution were filled into respective light-resistant brown ampoules.

Example 2

By following the same procedure as Example 1 using the same amount of mannitol in place of sorbitol, an injection was obtained.

Example 3

By following the same procedure as Example 1 using the same amount of xylitol in place of sorbitol, an injection was obtained.

Example 4

By following the same procedure as Example 1 using the same amount of propylene glycol in place of sorbitol, an injection was obtained.

Example 5

By following the same procedure as Example 1 using the same amount of glycerol in place of sorbitol, an injection was obtained.

Example 6

By following the same procedure as Example 1 using the same amount of inositol in place of sorbitol, an injection was obtained.

Example 7

By following the same procedure as Example 1 using 50 g of sorbitol, an injection was obtained.

Example 8

By following the same procedure as Example 1 using 100 g of sorbitol, an injection was obtained.

Example 9

By following the same procedure as Example 2 using 50 g of mannitol, an injection was obtained.

Example 10

By following the same procedure as Example 2 using 100 g of mannitol, an injection was obtained.

Example 11

By following the same procedure as Example 3 using 50 g of xylitol, an injection was obtained.

Example 12

By following the same procedure as Example 3 using 100 g of xylitol, an injection was obtained.

Example 13

By following the same procedure as Example 4 using 50 g of propylene glycol, an injection was obtained.

Example 14

By following the same procedure as Example 4 using 100 g of propylene glycol, an injection was obtained.

Example 15

About 2 litres of distilled water was heated to 50 to 60° C and 1.0 g of nicardipine hydrochloride and 125 g of sorbitol were dissolved therein with stirring. After cooling the solution (pH about 5.0) to room temperature, the pH of the solution was adjusted to 3.0 using 0.1 N hydrochloric acid. Then, the whole volume of the solution was adjusted to 2.5 litres by the addition of distilled water and after filtering the solution, 5 ml. aliquots of the solution were filled into respective light-resistant brown ampoules.

Example 16

By following the same procedure as Example 15, but adjusting the pH of the solution to 4.0, an injection was obtained.

Example 17

By following the same procedure as Example 15, but adjusting the pH of the solution to precisely 5.0 using 0.1 N sodium hydroxide in place of 0.1 N hydrochloric acid, an injection was obtained.

Example 18

By following the same procedure as Example 1 using 62.5 g of sorbitol and 62.5 g of mannitol in place of 125 g of sorbitol, an injection was obtained.

Comparison example

By following the same procedure as Example 15 but adjusting the pH of the solution to 2.0, an injection was obtained.

## Claims

1.  An injection of nicardipine hydrochloride comprising an aqueous nicardipine hydrochloride solution containing 2 to 7 w/v % of polyhydric alcohol and having a pH of 2.5 to 5.5.

2.  An injection according to claim 1 wherein the polyhydric alcohol is selected from sorbitol, mannitol, xylitol, propylene glycol, glycerol, and inositol.

3.  An injection according to claim 1 or 2 having a pH of 2.5 to 5.

4.  A process of producing a nicardipine hydrochloride injection which comprises forming a solution of nicardipine hydrochloride and 2 to 7 w/v% of polyhydric alcohol (based on the whole amount of the

EP 0 162 705 B1

injection) in water, the pH of the solution being 2.5 to 5.5.

5. A process according to claim 4 wherein the polyhydric alcohol is selected from sorbitol, mannitol, xylitol, propylene glycol, glycerol, and inositol.

6. A process according to claim 4 or 5 wherein the solution pH is 2.5 to 5, e.g. 3.5.

7. A process according to any of claims 4 to 6 which comprises dissolving the nicardipine hydrochloride and polyhydric alcohol in water, adjusting the solution pH, and then adding water to adjust the solution to a predetermined volume.


**Revendications**

1. Une solution injectable de chlorhydrate de nicardipine comprenant une solution aqueuse de chlorhydrate de nicardipine contenant de 2 à 7% p/v d'un polyol et ayant un pH compris entre 2,5 et 5,5.

2. Une solution injectable selon la revendication 1, dans laquelle le polyol est choisi parmi le sorbitol, le mannitol, le xylitol, le propylène-glycol, le glycérol et l'inositol.

3. Une solution injectable selon la revendication 1 ou 2, ayant un pH compris entre 2,5 et 5.

4. Un procédé de production d'une solution injectable de chlorhydrate de nicardipine qui comprend la formation d'une solution de chlorhydrate de nicardipine et de 2 à 7% p/v d'un polyol (basés sur la quantité totale de solution injectable) dans de l'eau, le pH de la solution étant compris entre 2,5 et 5,5.

5. Un procédé selon la revendication 4, dans lequel le polyol est choisi parmi le sorbitol, le mannitol, le xylitol, le propylène-glycol, le glycérol et l'inositol.

6. Un procédé selon la revendication 4 ou 5, dans lequel le pH de la solution est compris entre 2,5 et 5, par exemple de 3,5.

7. Un procédé selon l'une quelconque des revendications 4 à 6, qui comprend une dissolution du chlorhydrate de nicardipine et de du polyol dans de l'eau, l'ajustement du pH de la solution, et ensuite addition d'eau pour ajuster la solution à un volume prédéterminé.


**Ansprüche**

1. Injektion(slösung) von Nicardipin-hydrochlorid, umfassend eine wäßrige Nicardipin-hydrochlorid-Lösung, die 2 bis 7% (Gew./Vol.) eines mehrwertigen Alkohols enthält und einen pH-Wert von 2,5 bis 5,5 aufweist.

2. Injektionslösung nach Anspruch 1, wobei der mehrwertige Alkohol ausgewählt ist aus Sorbit, Mannit, Xylit, Propylenglykol, Glycerin und Inosit.

3. Injektionslösung nach Anspruch 1 oder 2 mit einem pH-Wert von 2,5 bis 5.

4. verfahren zur Herstellung einer Nicardipin-hydrochlorid-Injektionslösung, umfassend die Herstellung einer Lösung von Nicardipin-hydrochlorid und 2 bis 7% (Gew./Vol.) mehrwertigen Alkohol (bezogen auf die Gesamtmenge der Injektionslösung) in Wasser, wobei der pH-Wert der Lösung 2,5 bis 5,5 ist.

5. Verfahren nach Anspruch 4, wobei der mehrwertige Alkohol ausgewählt ist aus Sorbit, Mannit, Xylit, Propylenglykol, Glycerin und Inosit.

6. Verfahren nach Anspruch 4 oder 5, wobei der pH-Wert der Lösung 2,5 bis 5, z.B. 3,5, beträgt.

7. Verfahren nach einem der Ansprüche 4 bis 6, umfassend das Lösen des Nicardipin-hydrochlorids und

9

des mehrwertigen Alkohols in Wasser, Einstellen des pH-Werts der Lösung und anschließende Zugabe von Wasser, um die Lösung auf ein vorbestimmtes Volumen zu bringen.